# EUROPEAN PATENT SPECIFICATION

(11) **EP 1 700 583 B1**
(45) Date of publication and mention of the grant of the patent: **31.10.2007**
(21) Application number: 06101084.9
(22) Date of filing: 31.01.2006
(51) Int. Cl.: A61F 5/50

(54) **Device for preventing finger-sucking, particularly for babies**
Vorrichtung zum Verhindern des Fingerlutschens, besonders für Babys
Appareil pour empêcher la succion du doigt, en particulier pour le bébé

(30) Priority: 04.03.2005 ES 200500493 U
(43) Date of publication of application: 13.09.2006
(73) Proprietor: Cranc Solutions, S.L., 08035 Barcelona (ES)
(72) Inventor: Ibañez Gallego, Manel, 08035 Barcelona (ES)
(74) Representative: Ponti Sales, Adelaida

(56) References cited:
- DE-C- 461 057
- FR-A- 1 190 931
- US-A- 2 188 787
- US-A- 2 271 580
- US-A- 2 663 295
- US-A- 5 797 405
- US-B1- 6 283 126

## Description

The present invention relates to a device for preventing finger-sucking, particularly for babies.

### BACKGROUND OF THE INVENTION

Involuntary finger-sucking is a well-known problem, particularly as it relates to babies' thumbs, so that it is desirable to eradicate this bad habit as soon as possible.

Known in the art for avoiding this problem are devices that comprise a thimble-piece or sheath that covers the finger(s)/thumb(s) whose sucking is to be stopped, said sheath being attached to the hand or wrist by means of a tape or a glove that covers part of the hand. Thus, when the user moves the finger/thumb towards the mouth, the sheath prevents the finger/thumb being sucked.

Also known are rigid devices that are placed in the elbow zone to prevent the baby from bending its arm and therefore also prevent it from bringing its finger/thumb to its mouth.

Devices such as those described are nevertheless annoying to the user, as well as very eye-catching and aesthetically displeasing.

Patent US 6283126 A refers to a hand shield for a patient comprising an element of flexible but stiff material adapted to be rolled into an open ended generally fustro-conical shape. The element has a wrist wrap at the smaller end thereof. Means are provided for fastening the wrist wrap about the wrist of a patient. The element is sufficient long so as to extend beyond the fingers of the patient to prevent the fingers from grasping an object beyond an intermediate opening of said element.

### DESCRIPTION OF THE INVENTION

The object of the device of the present invention for preventing finger/thumb sucking, particularly for babies, is to solve the disadvantages presented by the devices known in the art, while providing a number of advantages that will be described below.

The device of the present invention for preventing finger-sucking, particularly for babies, is characterised in that it comprises an annular body, designed for placing around the baby's wrist and presenting a thickness in radial direction sufficient to stop the movement of the hand towards the mouth, due to the outer surface of said annular body coming into contact with the baby's face.

A device is thereby obtained to prevent finger-sucking, particularly for babies, which is very comfortable due to being worn like a bracelet, while it is of low cost and short production time because of being a very structurally simple device.

Advantageously, the annular body is manufactured from a flexible and light material, so as to avoid the baby hurting its face when it brings its hand to its mouth.

Preferably, the device of the invention includes means for opening and closing the annular body.

According to one embodiment of the invention, the opening and closing means include a transversal opening that allows the open ends of the annular body to be separated, thereby making it very easy to open and close.

Advantageously, the annular body includes an orifice provided to be fitted around the baby's wrist, having said orifice a shape largely coinciding with the outline of the baby's wrist, so as to ensure correct attachment of the device onto the wrist and thus prevent the device from moving or being removed by the baby.

### BRIEF DESCRIPTION OF THE DRAWINGS

In order to facilitate description of the aspects outlined above some drawings are attached that show, schematically and solely by way of non-restrictive for babies, in which:
Figure 1 is a schematic view of the device of the invention for preventing finger-sucking, fitted on the wrist of a baby;
Figure 2 is a plan view of the device of the invention; and
Figure 3 is a perspective view of the device of the invention.

### DESCRIPTION OF A PREFERRED EMBODIMENT

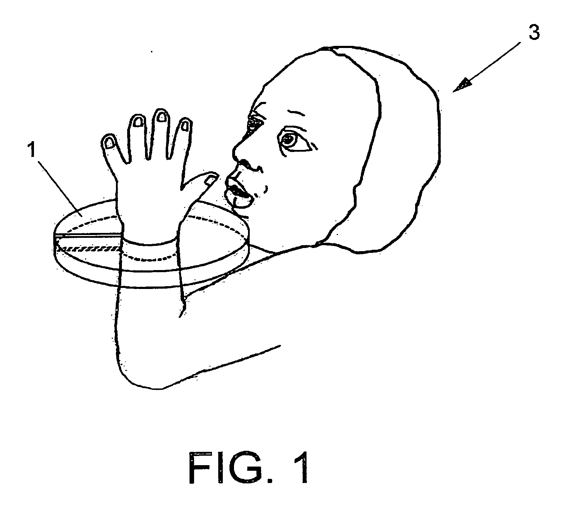

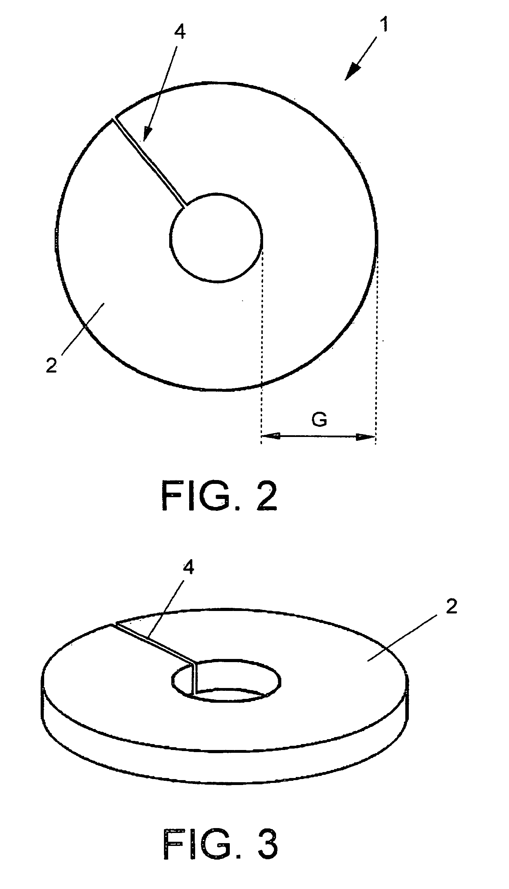

Figures 1 to 3 show a device 1 for preventing thumb-sucking in babies, which comprises an annular body 2 for placing around the baby's wrist 3 by way of a bracelet. Said annular body 2 has a thickness G in radial direction (see Figure 2) appropriate for halting the movement of bringing a hand to the mouth, due to the outer surface of said annular body 2 coming into contact with the baby's face 3. Said annular body 2 is manufactured from a flexible and light material to prevent the baby's face being injured when it raises its hand to its mouth.

The device 1 also includes means for opening and closing the annular body 2, which in this embodiment are made up of a transversal opening 4 that allows the open ends of the annular body 2 to be separated easily, thanks especially to the flexibility of the material.

The central orifice of the annular body presents a shape that largely coincides with the outline of the baby's wrist 3, in order to ensure correct attachment of the device 1 on the wrist and thereby prevent the device 1 from moving or being removed by the baby 3.

## Claims

1. Device (1) for preventing finger-sucking, particularly for babies, including an annular body (2), designed for placing around the baby's wrist (3), **characterised in that** it presents a thickness (G) in radial direction sufficient to stop the movement of the hand towards the mouth due to the outer surface of said annular body (2) coming into contact with the baby's face (3).

2. Device (1), according to claim 1, **characterised in that** the annular body (2) is manufactured from a flexible and light material.

3. Device (1), according to claims 1 or 2, **characterised in that** it includes means (4) for opening and closing the annular body (2).

4. Device (1), according to claim 3 **characterised in that** the opening and closing means include a transversal opening (4) that allows the open ends of the annular body (2) to be separated.

5. Device (1), according to any of the preceding claims, **characterised in that** the annular body (2) includes an orifice provided to be fitted around the baby's wrist (3), having said orifice a shape largely coinciding with the outline of the baby's wrist (3).

## Patentansprüche

1. Vorrichtung (1) zum Verhindern des Fingerlutschens, insbesondere für Babys, mit einem ringförmigen Körper (2) der ausgebildet ist für die Anordnung um das Handgelenk (3) des Babys, **dadurch gekennzeichnet, dass** sie eine Dicke (G) in radialer Richtung aufweist, die ausreichend ist, um die Bewegung der Hand zu dem Mund zu verhindern, dadurch, dass die äußere Oberfläche des ringförmigen Körpers (2) in Berührung mit dem Gesicht (3) des kommt.

2. Vorrichtung (1) nach Anspruch 1, **dadurch gekennzeichnet, dass** der ringförmige Körper (2) aus einem biegsamen und leichten Material hergestellt ist.

3. Vorrichtung (1) nach Anspruch 1 oder 2, **dadurch gekennzeichnet, dass** er eine Einrichtung (4) zum Öffnen und Schließen des ringförmigen Körpers (2) umfasst.

4. Vorrichtung nach Anspruch 3, **dadurch gekennzeichnet, dass** die Einrichtung zum Öffnen und Schließen eine Queröffnung (4) umfasst, die ermöglicht, dass die offenen Enden des ringförmigen Körpers (2) getrennt werden.

5. Vorrichtung (1) nach einem der vorhergehenden Ansprüche, **dadurch gekennzeichnet, dass** der ringförmige Körper (2) eine Öffnung aufweist, die vorgesehen ist, um das Handgelenk (3) des Babys gesetzt zu werden, wobei die Öffnung eine Form aufweist, die weitgehend mit der Kontur des Handgelenks (3) des Babys übereinstimmt.

## Revendications

1. Dispositif (1) pour empêcher de sucer un doigt, destiné en particulier à des bébés, comportant un corps annulaire (2), conçu pour être placé autour du poignet du bébé (3), **caractérisé en ce qu'**il présente une épaisseur (G) dans la direction radiale suffisante pour arrêter le mouvement de la main vers la bouche du fait que la surface extérieure dudit corps annulaire (2) vient en contact avec la face du bébé (3).

2. Dispositif (1) selon la revendication 1, **caractérisé en ce que** le corps annulaire (2) est fabriqué à partir d'un matériau souple et léger.

3. Dispositif (1) selon la revendication 1 ou 2, **caractérisé en ce qu'**il comporte des moyens (4) pour ouvrir et fermer le corps annulaire (2).

4. Dispositif (1) selon la revendication 3, **caractérisé en ce que** les moyens d'ouverture et de fermeture comportent une ouverture transversale (4) qui permet de séparer les extrémités ouvertes du corps annulaire (2).

5. Dispositif (1) selon l'une quelconque des revendications précédentes, **caractérisé en ce que** le corps annulaire (2) comporte un orifice prévu pour être agencé autour du poignet du bébé (3), ledit orifice ayant une forme coïncidant de manière ample avec le contour du poignet du bébé (3).
